# EUROPÄISCHE PATENTSCHRIFT

(11) **EP 1 444 194 B1**
(45) Veröffentlichungstag und Bekanntmachung des Hinweises auf die Patenterteilung: **09.12.2009**
(21) Anmeldenummer: 02781275.9
(22) Anmeldetag: 22.10.2002
(51) Int. Cl.: C07C 229/12, C07K 7/64

(54) **N-METHYL-HOMOCYSTEINE UND IHRE VERWENDUNG SOWIE VERFAHREN ZU IHRER HERSTELLUNG**
N-METHYL-HOMOCYSTINES, USE THEREOF AND METHOD FOR THE PRODUCTION THEREOF
N-METHYLHOMOCYSTEINES, LEUR UTILISATION ET LEUR PROCEDE DE PRODUCTION

(30) Priorität: 15.11.2001 US 331416 P; 28.03.2002 DE 10215336
(43) Veröffentlichungstag der Anmeldung: 11.08.2004
(73) Patentinhaber: CIS bio international, 91192 Gif sur Yvette Cedex (FR)
(72) Erfinder: WILLUHN, Marc, 10439 Berlin (DE); PLATZEK, Johannes, 12621 Berlin (DE); OTTOW, Eckhard, 12203 Berlin (DE); PETROV, Orlin, 14199 Berlin (BG); BORM, Claudia, 10559 Berlin (DE); HINZ, Dirk, 13509 Berlin (DE); MANN, Gregor, 12159 Berlin (DE); LISTER-JAMES, John, Bedford, NH 03110 (US); WILSON, David, M., Bow, NH 03304 (US)
(74) Vertreter: Eder, Michael
(86) Internationale Anmeldenummer: PCT/EP2002/011779
(87) Internationale Veröffentlichungsnummer: WO 2003/042163

(56) Entgegenhaltungen:
- US-B1- 6 333 410
- R. M. FREIDINGER ET AL: "Synthesis of 9-Fluorenylmethydroxycarbonyl-Proctected N-Alkyl Amino Acids by Reduction of Oxazolidinones" JOURNAL OF ORGANIC CHEMISTRY, Bd. 48, 1983, Seiten 77-81, XP002235793 in der Anmeldung erwähnt

## Beschreibung

Die Erfindung betrifft die in den Patentansprüchen gekennzeichneten Gegenstände; nämlich N-Methyl-Homocysteine der allgemeinen Formel I und II, ihre Verwendung und Verfahren zu ihrer Herstellung.

N-Methyl-Aminosäuren sind wertvolle Zwischenprodukte in der Synthesechemie. Vor allem die pharmazeutische Chemie verwendet derartige Bausteine sehr häufig, da viele hochpotente und selektive Pharmaka N-Methyl-Aminosäuren enthalten.

Optisch aktive N-Methyl-Aminosäuren kommen als Einzelverbindungen natürlich vor (z. B. N-Methyl-Tryptophane: Liebigs Ann. Chem. 1935, 520, 31-34), finden sich aber auch als Bausteine in einer Reihe von biologisch aktiven Naturstoffen wie z. B. den Dolastatinen (G. R. Pettit et al., J. Org. Chem. 1990, 55, 2989-2990; Tetrahedron 1993, 41, 9151-9170) oder den Didemninen (K. L. Rinehart et al., J. Nat. Prod. 1988, 51, 1-21; J. Am. Chem. Soc. 1987, 109, 6846-6848; J. Am. Chem. Soc. 1995, 117, 3734-3748). Weitere Beispiele sind Jasplakinolid (J. Org. Chem. 1991, 56, 5196-5202) und andere cytotoxische Peptide (J. Org. Chem. 1989, 54, 617-627; Tetrahedron 1995, 51, 10653-10662; J. Org. Chem. 1989, 54, 736-738). Auch das immunosuppressiv wirkende Cyclosporin enthält N-Methyl-Aminosäuren (siehe beispielsweise R. M. Wenger, Heilv. Chim. Acta 1983, 66, 2672-2702; S. L. Schreiber et al., Tetrahedron Lett. 1988, 29, 6577-6580). N-Methyl-Aminosäuren können auch neuropharmakologische Aktivität besitzen (J. C. Watkins, J. Med. Pharm. Chem. 1962, 5, 1187-1199).

Der Einbau von N-Methyl-Aminosäuren in biologisch aktive Peptide wird zum einen in der pharmazeutischen Forschung oft genutzt, um Konformation und biologische Aktivität zu untersuchen (siehe z. B. J. Org. Chem. 1981, 46, 3436-3440; Int. J. Pept. Protein Res. 1995, 46, 47-55; Int. J. Pept. Protein Res. 1986, 27, 617-632). Einen Überblick über Konformationsuntersuchungen an Peptiden und den Zusammenhang mit biologischer Aktivität geben H. Kessler, Angew. Chem. 1982, 94, 509-520 und G. R. Marshall et al., Ann. Rep. Med. Chem. 1978, 13, 227-238.

Zum anderen dient die Substitution von Aminosäuren in Peptiden durch die entsprechenden N-Methyl-Verbindungen dazu, die Wirksamkeit oder Selektivität von Peptidliganden zu erhöhen (siehe z. B. J. Med. Chem. 1994, 37, 769-780; Int. J. Pept. Protein Res. 1995, 46, 47-55). N-Methyl-Peptidbindungen zeigen oft höhere Stabilität gegenüber Proteolyse als die nicht-methylierten, was zu einer erhöhten oralen Verfügbarkeit und verlängerten Wirksamkeit führen kann (R. H. Mazur et al., J. Med. Chem. 1980, 23, 758-763). Zum Design von Peptiden durch Einbau von N-Methyl-Aminosäuren und durch andere Modifikationen siehe W. F. Degrado, Adv. Protein Chem. 1988, 39, 51-124 und J. Rizo, L. M. Gierasch, Annu. Rev. Biochem. 1993, 61, 387-418. Für Übersichten zu Peptidomimetika siehe A. Giannis et al., Angew. Chem. 1993, 105, 1303-1326 und J. Gante, Angew. Chem. 1994, 106, 1780-1802.

In US 6,333,410 wird ein Verfahren zur Herstellung und Aufreinigung von thiol-enthaltenden Maytansinoiden beschrieben. Ein Intermediat dieses Verfahres ist ein S-geschütztes. N-monosubstituiertes Methionin. US 6,333,410 offenbart jedoch keine S-qeschützten, N,N-disubstituierten Methionine.

Feidinger at al. (J. Org. Chem., 1983) beschreibt Verfahren zur Herstellung von 9-fluorenylmethyloxycarbonyl-geschützten N-alkyl Aminosäuren durch Reduktion von Oxazolidinonen. Eines der dabei verwendeten Edukte ist N-monosubstituiertes Methionin. S-geschützte, N,N-disubstituierte Methionine werden in Feidinger nicht erwähnt.

In WO 01/44177 ist ein Peptid beschrieben, das in einer cyclischen 6-mer Teilsequenz ein N-Methyl-Homocystein enthält. Über das Schwefelatom des Homocysteins erfolgt die Anbindung einer Metallionen-bindenden Sequenz. Durch die Chelatisierung von Radionukliden wie ^{99m}Tc bzw. ¹⁸⁸Re ist der Einsatz als Radiodiagnostika bzw. Radiotherapeutika in der Diagnose bzw. Therapie von Krebserkrankungen möglich (siehe auch US 6,056,940; US 6,022,857; US 6,017,509).

Die Synthese des Peptids erfolgt in WO 01/44177 durch N-Methylierung im Verlauf der stufenweisen Synthese (siehe z. B. J. Am. Chem. Soc. 1997, 119, 2301-2302). Ein 4-mer Peptid, das C-terminal an eine feste Phase gebunden ist, wird mit der Aminosäure N-Fmoc-S-tritylhomocystein (Fmoc = 9H-Fluoren-9-ylmethyloxycarbonyl), die in drei Schritten aus Methionin zugänglich ist (J. Med. Chem. 1996, 39, 1361-1371), zum 5-mer Peptid umgesetzt. Nach Abspaltung der Schutzgruppe wird die terminale Aminogruppe des Homocysteins mit 2-Nitrobenzolsulfonsäurechlorid zum Sulfonamid umgesetzt. Dann erfolgt die Methylierung mit MTBD (1,3,4,6,7,8-Hexahydro-1-methyl-2H-pyrimido[1,2-a]pyrimidin) und nachfolgend die Abspaltung der Sulfonylgruppe mit 2-Mercaptoethanol. Dann wird die Peptidsequenz weiter aufgebaut. Diese Synthese hat jedoch den Nachteil einer unvollständigen Umsetzung und erfordert eine zusätzliche Aufreinigung des Peptids.

Der Aufbau von Peptidsequenzen, die N-Methyl-Aminosäuren enthalten, erfolgt üblicherweise durch Umsetzung einer Teilsequenz mit einer Fmoc-geschützten N-Methyl-Aminosäure (siehe z. B. J. Med. Chem. 1996, 39, 1361-1371). Die für die Peptidsequenz in WO 01/44177 benötigte Aminosäure N-Fmoc-N-Methyl-S-tritylhomocystein konnte jedoch bisher nicht hergestellt werden.

Es gibt in der Literatur eine Reihe von Methoden zur Darstellung von N-Methyl-Aminosäuren, die üblicherweise von den entsprechenden nicht-methylierten Aminosäuren ausgehen, die als chirale Naturstoffe in großer Menge kommerziell erhältlich sind.

Die erste Synthese nicht-racemischer N-Methyl-Aminosäuren geht zurück auf E. Fischer. In diesem Fall werden die N-Tosylderivate der Aminosäuren methyliert durch Umsetzung mit Natronlauge und Methyliodid. Daran anschließend erfolgt die Abspaltung der Tosylgruppe mit siedender Salzsäure (Ann. Chem. 1913, 398, 96-125; Ber. dt. chem. Ges. 1915, 48, 360-378). Es ist beschrieben, dass bei dieser Methode teilweise Racemisierung auftritt (A. H. Cook et al., J. chem. Soc. 1949, 1022-1028).

Eine weitere Methode von Fischer ist die Umsetzung von 2-Bromcarbonsäuren mit Methylamin (Ber. dt. chem. Ges. 1916, 49, 1355-1366; für eine Anwendung siehe auch A. H. Cook et al., J. chem. Soc. 1949, 1022-1028). Auch dabei entstehen teilweise racemisierte Produkte (siehe P. Quitt et al., Helv. Chim. Acta 1963, 46, 327-333). Wir konnten diese Beobachtung bei einer Umsetzung von 2-Brom-4-methylsulfanyl-butansäure mit Methylamin bestätigen. In der Literatur ist die Darstellung von N-Methyl-L-methionin beschrieben über die Diazotierung von D-Methionin mit anschließender Substitution durch Bromid unter Retention, gefolgt von einer Substitution durch Methylamin unter Inversion (N. Izumiya, A. Nagamatsu, Kyushu. Mem. Med. Sci. 1953, 4, 1-16). Die Enantiomerenreinheit des Produkts wurde nicht bestimmt. Wir konnten zeigen, dass auch mit einer als milde beschriebenen Diazotierungsmethode von Ellman (Synthesis 1999, 583-585) die Reaktionssequenz nur mit 75% Enantiomerenüberschuss abläuft. Diese Methode ist daher für die Darstellung von enantiomerenreinen N-Methyl-Homocysteinen nicht geeignet.

Eine sehr häufig eingesetzte Methode ist die direkte Alkylierung nach Benoiton mit Natriumhydrid und Methyliodid in THF oder DMF (Can. J. Chem. 1973, 51, 1915-1919; Can. J. Chem. 1977, 55, 906-910). Diese Methode wird standardmäßig verwendet zur Darstellung von Z- und Boc-geschützten N-Methyl-Aminosäuren (siehe z. B. Tetrahedron 1995, 51, 10653-10662; D. L. Boger et al., J. Am. Chem. Soc. 1999, 121, 6197-6205; H. Waldmann et al., Chem. Eur. J. 1999, 5, 227-236). Die Umsetzung von Boc-geschütztem Methioninamid mit Methylierungsmitteln wie Methyliodid führt in unserem Fall zur Methylierung des Schwefelatoms unter Bildung von Sulfoniumionen (siehe auch S. J. F. Macdonald et al., J. Med. Chem. 1999, 64, 5166-5175). Diese Methode ist daher für die Darstellung von N-Methyl-Homocysteinen nicht anwendbar.

Es gibt eine Reihe von Methoden zur Darstellung von N-Methyl-Aminosäuren, die von Aminosäureestern ausgehen. Dazu zählen die selektive Reduktion von N-Formyl Aminosäureestern mit Boran (Tetrahedron Lett. 1982, 23, 3315-3318; J. Org. Chem. 1991, 56, 5196-5202) und die Umsetzung von Schiffschen Basen von Aminosäureestern mit Dimethylsulfat oder Methyltriflat mit nachfolgender Hydrolyse (M. J. O'Donnell et al., Tetrahedron Lett. 1984, 25, 3651-3654). Bei der Verseifung von Aminosäureestern zu Aminosäuren tritt jedoch teilweise Racemisierung auf (S. T. Cheung, N. L. Benoiton, Can. J. Chem. 1977, 55, 906-910). Daher sind diese Methoden ebenfalls nicht geeignet.

Aminosäuren können ebenfalls in einer reduktiven Alkylierung mit einem Aldehyd umgesetzt werden. Die intermediär gebildete Schiffsche Base wird in situ in Gegenwart von Wasserstoff und eines Katalysators (R. E. Bowman et al. J. Chem. Soc. 1950, 1342-1345 und 1346-1349) oder mit Natriumborhydrid (K. A. Schellenberg, J. Org. Chem. 1963, 28, 3259-3261) reduziert. Diese Methode wird sowohl in Lösung (siehe z. B. J. Org. Chem. 1996, 61, 3849-3862; J. Org. Chem. 1995, 60, 6776-6784) als auch an fester Phase (siehe z. B. J. Org. Chem. 1996, 61, 6720-6722; Tetrahedron Lett. 1997, 38, 4943-4946; Bioorg. Med. Chem. Lett. 1995, 5, 47-50) durchgeführt. Schon von Bowman wurden auch Di- und Tripeptide nach dieser Methode permethyliert (J. Chem. Soc. 1950, 1349-1351). Eine selektive Monomethylierung mit Formaldehyd ist nach dieser Methode nicht möglich, sondern führt zu Gemischen aus unmethylierter, monomethylierter und dimethylierter Aminosäure, die sich nicht auftrennen lassen (Quitt et al., Helv. Chim. Acta 1963, 46, 327-333; siehe auch R. E. Bowman, H. H. Stroud, J. Chem. Soc. 1950, 1342-1345).

Für die Synthese von Fmoc-geschützten N-Methyl-Aminosäuren erfreut sich die Methode von Freidinger (J. Org. Chem. 1983, 48, 77-81) großer Beliebtheit. Diese Methode besteht aus zwei Stufen: Im ersten Schritt wird durch Umsetzung der Aminosäure mit Formaldehyd ein Oxazolidinon gebildet, das dann im zweiten Schritt mittels Triethylsilan unter sauren Bedingungen zur N-Methylverbindung reduziert wird. Von Freidinger wurde die Umsetzung von Fmoc-L-Methionin mit Formaldehyd zum Oxazolidinon mit 88 % Ausbeute beschrieben, doch die Reduktion mit Triethylsilan zu Fmoc-N-methyl-L-methionin ist selbst nach fünf Tagen noch unvollständig und liefert nur 22 % Ausbeute (J. Org. Chem. 1983, 48, 77-81).

Aufgabe der Erfindung war es daher, Verbindungen zu finden, die zum Aufbau von Peptidsequenzen, die N-methylierte Homocysteine enthalten, dienen und die N-Methylierung an fester Phase vermeiden, sowie eine Methode zu deren Herstellung.

Die Aufgabe der Erfindung wird durch die N-Methyl-Homocysteine, die sowohl enantiomerenrein als auch racemisch sein können, der allgemeinen Formel I gelöst, 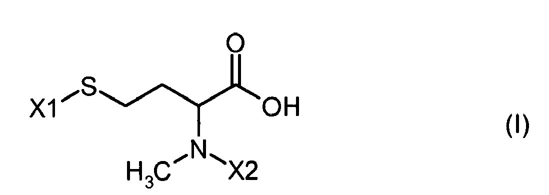 worin X1 für eine Schwefel-Schutzgruppe und X2 für ein Wasserstoffatom oder für eine Stickstoff-Schutzgruppe steht. Vorzugsweise steht X1 für Benzyl (Bn), 4-Methoxybenzyl (Mob), Diphenylmethyl, Bis(4-methoxyphenyl)methyl, 4,4'-Dimethoxytriphenylmethyl (DMT), Triphenylmethyl (Trityl), Methoxymethyl (MOM), 9H-Fluoren-9-ylmethyl oder tert-Butylsulfid (T. W. Greene, P. G. M. Wuts, Protective Groups in Organic Synthesis, 2nd edition, J. Wiley & Sons, New York 1991), bevorzugt für Benzyl (Bn), 4-Methoxybenzyl (Mob), Diphenylmethyl, Bis(4-methoxyphenyl)methyl, 4,4'-Dimethoxytriphenylmethyl (DMT) oder Triphenylmethyl (Trityl), besonders bevorzugt für Triphenylmethyl (Trityl), und X2 im Falle einer Schutzgruppe vorzugsweise für 9H-Fluoren-9-ylmethyloxycarbonyl (Fmoc), 2,7-Dibrom-9H-fluoren-9-ylmethyloxycarbonyl, tert-Butyloxycarbonyl (Boc), Benzyloxycarbonyl (Cbz o. Z), Trifluoracetyl, 2,2,2-Trichloroethyloxycarbonyl (Troc), 2-Trimethylsilylethyloxycarbonyl (Teoc) oder 2-Trimethylsilylethylsulfonyl (T. W. Greene, P. G. M. Wuts, s. o.), bevorzugt für tert-Butyloxycarbonyl (Boc), Benzyloxycarbonyl (Cbz o. Z) oder 9H-Fluoren-9-ylmethyloxy-carbonyl (Fmoc), besonders bevorzugt für 9H-Fluoren-9-ylmethyloxycarbonyl (Fmoc).

Ein Aspekt der Erfindung betrifft N-Methyl-Homocysteine, die sowohl enantiomerenrein als auch racemisch sein können, der allgemeinen Formel I worin X1 für eine Schwefel-Schutzgruppe und X2 für eine Stickstoff-Schutzgruppe steht.

Die Herstellung der erfindungsgemäßen Verbinden der allgemeinen Formel I erfolgt dadurch, dass das Oxazolidinon der Formel II, 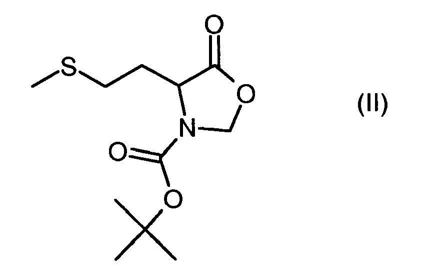 das nach Literaturmethoden (Ben-Ishai, J. Am. Chem. Soc. 1957, 79, 5736-5738; M. A. Blaskovich, M. Kahn, Synthesis 1998, 379-380; G. V. Reddy et al., Synth. Commun. 1999, 29, 4071-4077) aus Methionin hergestellt wird (siehe Beispiel 1), mit einem Reduktionsmittel in Gegenwart von Säure zum N-Methylmethionin der Formel III 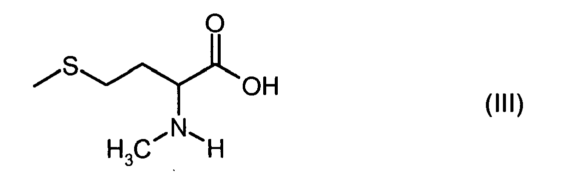 geöffnet wird und anschließend in an sich bekannter Weise (siehe T. W. Greene, P. G. M. Wuts, s. o.) eine Schwefel-Schutzgruppe sowie gegebenenfalls eine Stickstoff-Schutzgruppe eingeführt werden. Vorzugsweise werden als Reduktionsmittel Alkylsilane verwendet, bevorzugt Triethylsilan. Vorzugsweise werden als Säuren Trifluoressigsäure, Pentafluorpropionsäure, Trifluormethansulfonsäure oder Methansulfonsäure verwendet, bevorzugt Trifluoressigsäure. Vorzugsweise erfolgt die Reaktion in chlorierten Lösungsmitteln, Dioxan, THF, Dimethoxyethan, bevorzugt in Dichlormethan oder Chloroform. Die Reaktion wird bei einer Temperatur von -20 °C bis +100 °C durchgeführt, vorzugsweise bei 0 bis 40 °C. Die Reaktionszeiten liegen bei fünf Minuten bis zehn Stunden, vorzugsweise bei 0.5 bis 2 h. N-Methylmethionin wird mit Ausbeuten von 70 bis 95 % erhalten.

Die Verbindungen der allgemeinen Formel Iwerden nach dem Fachmann bekannten Methoden (siehe M. Gooodman (Hrsg.), Houben-Weyl, Vol. E22, Synthesis of Peptids and Peptidomimetics, Thieme, 2001) verwendet zum Aufbau von N-Methyl-Homocystein enthaltenden Peptiden und Peptidzwischenstufen, vorzugsweise zur Herstellung von Fmoc-(N-CH₃)Hcy(Trt)-Tyr(tBu)-D-Trp(Boc)-Lys(Boc)-Thr(tBu)-OH, cyclo-Tyr-D-Trp-Lys-Thr-Phe-(N-CH₃)Hcy und cyclo-Tyr-D-Trp-Lys-Thr-Phe-(N-CH₃)Hcy(CH₂CO-β-Dap-Phe(4-NH₂)-Cys-Thr-Ser).

### Beispiel 1

### a) tert-Butyl-(S)-4-[2-(methylsulfanyl)ethyl)]-5-oxooxazolidinon-3-carboxylat

Eine Mischung von 100 g N-(tert-Butoxycarbonyl)-L-methionin (0.4 mol), 100 g Paraformaldehyd (3.3 mol), 200 g getrocknetem Magnesiumsulfat (1.7 mol) und 4 g Paratoluolsulfonsäure (0.02 mol) in 1 I Toluol wird für 3 Stunden auf 90 °C erhitzt. Man lässt auf 20 °C abkühlen und gibt unter Eiskühlung 800 ml einer gesättigten Natriumhydrogencarbonatlösung dazu. Es wird abfiltriert und der Rückstand mit 400 ml Ethylacetat gewaschen. Die organische Phase wird mit 300 ml Wasser extrahiert, über Natriumsulfat getrocknet und im Vakuum zur Trockne eingeengt. Das Rohprodukt wird in 150 ml Hexan/Ethylacetat 1:3 gelöst und über Kieselgel filtriert und das Kieselgel mit 500 ml Hexan/Ethylacetat 1:3 nachgewaschen. Man engt im Vakuum zur Trockne ein und erhält 85.3 g (0.33 mmol, 83 % der Theorie) eines gelben Öls.

| | | | | |
|---|---|---|---|---|
| Ber.: | C 50.56 | H 7.33 | N 5.36 | S 12.27 |
| Gef.: | C 50.38 | H 7.24 | N 5.43 | S 12.10 |

IR: 2980, 2920, 1800, 1715, 1390, 1170, 1050
NMR (CDCl3): 1.5 (9H), 2.1 (3H), 2.15 - 2.35 (2H), 2.5 - 2.68 (2H), 4.33 (1H), 5.21 (1H), 5.48 (1H)
MS (EI): m/z 261, 205, 188, 116, 100, 57

### b) N-Methyl-L-methionin

Zu einer Lösung von 19.4 g tert-Butyl-(S)-4-[2-(methylsulfanyl)ethyl)]-5-oxooxazolidinon-3-carboxylat (0.074 mol) in 65 ml Dichlormethan werden bei 0 °C 45 ml Trifluoressigsäure und 40 ml Triethylsilan (0.25 mol) getropft. Es wird 1 Stunde unter Eiskühlung und 1 Stunde bei 20 °C nachgerührt. Anschließend wird die Reaktionslösung im Vakuum zur Trockne eingeengt. Man nimmt den Rückstand dreimal mit 100 ml Dichlormethan wieder auf und engt erneut zur Trockne ein. Dann nimmt den Rückstand in 100 ml Wasser auf und extrahiert dreimal mit 50 ml MTBE. Die Wasserphase wird im Vakuum zur Trockne eingeengt. Man nimmt den Rückstand dreimal mit 50 ml Ethanol wieder auf und engt erneut zur Trockne ein. Der Rückstand wird mit 150 ml MTBE versetzt und für 2 Stunden bei 20 °C gerührt. Man filtriert ab und wäscht mit 50 ml MTBE nach. Nach Trocknen erhält man 9.6 g (0.059 mol, 80 % d. Th.) eines farblosen Feststoffs.

| | | | | |
|---|---|---|---|---|
| Ber.: | C 44.15 | H 8.03 | N 8.58 | S 19.64 |
| Gef.: | C 44.01 | H7 .83 | N 8.72 | S 19.80 |

IR: 3440, 3010, 2920, 2830, 2420, 1735, 1675, 1580, 1205, 1140, 800, 720 NMR (DMSO): 2.01 - 2.08 (5H), 2.5 - 2.65 (5H), 3.74 - 3.78 (1H)
MS (FAB): m/z 164
CD (Wasser): 200 nm, Δε 1.64

### c) N-Methyl-S-trityl-L-homocystein

Man legt 7.5 g (0.046 mol) N-Methyl-L-methionin vor und kondensiert unter MeOH/Trockeneis-Kühlung 200 ml Ammoniak ein. Bei -35°C werden portionsweise über eine Stunde 5.2 g (0.23 mol) Natrium zugegeben und zwei Stunden nachgerührt. Dann werden portionsweise 9.7 g (0.18 mol) Ammoniumchlorid zugegeben, die Kühlung entfernt und über Nacht mit Stickstoff der Ammoniak ausgetrieben. Man gibt 14.0 g (0.054 mol) Triphenylmethanol zu. Unter Eiskühlung werden dann 50 ml Dichlormethan und 90 ml Trifluoressigsäure zugegeben. Man rührt eine Stunde bei RT und engt zur Trockne ein. Der Rückstand wird in 200 ml Wasser suspendiert und mit Natronlauge auf pH 13 gebracht. Nach einer Stunde Nachrühren saugt man ab und suspendiert den Feststoff in 500 ml Wasser. Durch Zugabe von Citronensäure wird auf pH 4 eingestellt. Es werden 600 ml MTBE zugegeben und 30 Minuten gerührt. Man saugt ab und wäscht zweimal mit je 100 ml MTBE. Der Rückstand wird mit 100 ml Dichlormethan versetzt, 20 ml Ethanol zugegeben und anschließend 500 ml MTBE zugetopft. Eine Stunde nachgerührt, abgesaugt, mit MTBE gewaschen. Nach Trocknen erhält man 12.6 g (0.032 mol, 70 % d. Th.) eines farblosen Feststoff.

| | | | | |
|---|---|---|---|---|
| Ber.: | C 73.62 | H 6.44 | N 3.58 | S 8.19 |
| Gef.: | C 73.40 | H 6.30 | N 3.71 | S 8.02 |

IR: 3440, 3055, 2850, 2400, 1615, 1485, 1445, 1395, 740, 700
NMR (CDCl3): 1.28 - 1.62 (2H), 2.12(3H), 2.25 - 2.45 (2H)2.84 - 2.92 (1H), 7.12 - 7.41 (15H)
MS (EI): m/z 243, 165

### d) N-(9H-Fluoren-9-ylmethyloxycarbonyl)-N-methyl-S-trityl-L-homocystein

16.6 g (0.042 mol) N-Methyl-S-trityl-L-homocystein werden in 90 ml Wasser und 100 ml THF suspendiert und 12.58 g (0.12 mol) Natriumcarbonat zugegeben. Bei 10 °C werden 14.88 g (0.044 mol) Fluorenylmethylsuccinimidylcarbonat in 50 ml THF zugetropft und drei Stunden nachgerührt. Dann wird mit 80 ml Wasser und 130 ml Ethylacetat versetzt, zehn Minuten gerührt und mit Citronensäure auf pH 4 gebracht. Die organische Phase wird zweimal mit je 100 ml Wasser und einmal mit 100 ml Kochsalzlösung gewaschen. Die wäßrigen Phasen werden einmal mit 100 ml Ethylacetat nachextrahiert. Die vereinigte organische Phase wird am Rotationsverdampfer eingedampft, mit 260 ml Ethylacetat versetzt und am Rotationsverdampfer zu 29.2 g Schaum eingedampft, der mit Dichlormethan/Aceton 6:4 chromatographiert wird. Man erhält 22.4 g (0.036 mol, 86 % d. Th.) Produkt.

| | | | | |
|---|---|---|---|---|
| Ber.: | C 76.32 | H 5.75 | N 2.28 | S 5.22 |
| Gef.: | C 76.11 | H 5.52 | N 2.40 | S 5.38 |

IR: 3430, 3060, 2950, 1740, 1710, 1175, 740, 700
NMR (CDCl3): 1.46 - 2.35 (4H), 2.66 - 2.73 (3H), 4.13 - 4.6 (4H), 7.13 - 7.79 (23H) MS (FAB): m/z 614, 636

### Beispiel 2

Die zu der Titelverbindung von Beispiel 1 d enantiomere Verbindung kann in analoger Weise erhalten werden, wenn man wie oben beschrieben anstelle von N-(tert-Butoxycarbonyl)-L-methionin N-(tert-Butoxycarbonyl)-D-methionin verwendet.

### a) tert-Butyl-(R)-4-[2-(methylsuIfanyl)ethyl)]-5-oxooxazolidinon-3-carboxylat

| | | | | |
|---|---|---|---|---|
| Ber.: | C 50.56 | H 7.33 | N 5.36 | S 12.27 |
| Gef.: | C 50.30 | H 7.51 | N 5.50 | S 12.41 |

IR: 2980, 2920, 1800, 1715, 1390, 1175, 1050
NMR (CDCl3): 1.5 (9H), 2.08 (3H), 2.15 - 2.35 (2H), 2.5 - 2.68 (2H), 4.33 (1H), 5.21 (1H), 5.48 (1H)
MS (EI): m/z 261, 205, 188, 116, 100, 57

### b) N-Methyl-D-methionin

| | | | | |
|---|---|---|---|---|
| Ber.: | C 44.15 | H 8.03 | N 8.58 | S 19.64 |
| Gef.: | C 43.98 | H 7.81 | N 8.70 | S 19.82 |

IR: 3430, 3010, 2920, 2830, 2420, 1630, 1580, 1395
NMR (D2O): 2.13 (3H), 2.12 - 2.22 (2H), 2.55 - 2.68 (2H), 2.74 (3H), 3.68 - 3.73 (1H)
MS (EI): m/z 163, 145, 118, 88, 70, 61
CD (Wasser): 200 nm, Δε 1.87

### c) N-Methyl-S-trityl-D-homocystein

| | | | | |
|---|---|---|---|---|
| Ber.: | C 73.62 | H 6.44 | N 3.58 | S 8.19 |
| Gef.: | C 73.45 | H 6.25 | N 3.78 | S 8.15 |

IR: 3440, 3055, 2850, 2400, 1615, 1485, 1445, 1395, 740, 700
NMR (CDCl3): 1.28 -1.62 (2H), 2.12(3H), 2.25 - 2.45 (2H), 2.84 - 2.92 (1H), 7.12 - 7.41 (15H)
MS (EI): m/z 243, 165

### d) N-(9H-Fluoren-9-ylmethyloxycarbonyl)-N-methyl-S-trityl-D-homocystein

| | | | | |
|---|---|---|---|---|
| Ber.: | C 76.32 | H 5.75 | N 2.28 | S 5.22 |
| Gef.: | C 76.15 | H 5.58 | N 2.41 | S 5.30 |

IR: 3440, 3055, 2950, 2600, 1740, 1705, 1445, 1170, 740, 700
NMR (CDCl3): 1.45 - 2.35 (4H), 2.65 - 2.73 (3H), 4.14 - 4.62 (4H), 7.13 - 7.81 (23H)
MS (FAB): m/z 636, 614

### Beispiel 3

### N-Benzyloxycarbonyl-N-methyl-S-trityl-L-homocystein

16.6 g (0.042 mol) N-Methyl-S-trityl-L-homocystein (Titelverbindung aus Beispiel 1 c) werden in 90 ml Wasser und 100 ml THF suspendiert und 12.58 g (0.12 mol) Natriumcarbonat zugegeben. Bei 10 °C werden 10.97 g (0.044 mol) N-Benzyloxycarbonyloxysuccinimid gelöst in 50 ml THF zugetropft und drei Stunden bei 10°C nachgerührt. Dann wird mit 80 ml Wasser und 130 ml Ethylacetat versetzt, zehn Minuten bei Raumtemperatur gerührt und mit Citronensäure auf pH 4 gebracht. Die organische Phase wird zweimal mit je 100 ml Wasser und einmal mit 100 ml Kochsalzlösung gewaschen. Die wäßrigen Phasen werden einmal mit 100 ml Ethylacetat nachextrahiert. Die vereinigten organischen Phasen werden im Vakuum zur Trockene eingedampft ; der Rückstand wird an Kieselgel chromatographiert (Laufmittel : Dichlormethan/Aceton 6:4) . Man erhält 22.08 g ( 87 % der Theorie ) Produkt als festen, farblosen Schaum.

| Elementaranalyse : | | | | |
|---|---|---|---|---|
| Ber.: | C 73.12 | H 5.94 | N 2.66 | S 6.10 |
| Gef.: | C 72.87 | H 6.08 | N 2.60 | S 5.98 |

### Beispiel 4

### N-tert-Butyloxycarbonyl-N-methyl-S-trityl-L-homocystein

16.6 g (0.042 mol) N-Methyl-S-trityl-L-homocystein (Titelverbindung aus Beispiel 1 c) werden in 90 ml Wasser und 100 ml THF suspendiert und 12.58 g (0.12 mol) Natriumcarbonat zugegeben. Bei 10 °C werden 9.60 g (0.044 mol) Di-tert.-Butyl-dicarbonat, gelöst in 50 ml THF zugetropft und drei Stunden bei 10°C nachgerührt. Dann wird mit 80 ml Wasser und 130 ml Ethylacetat versetzt, zehn Minuten bei Raumtemperatur gerührt und mit Citronensäure auf pH 4 gebracht. Die organische Phase wird zweimal mit je 100 ml Wasser und einmal mit 100 ml Kochsalzlösung gewaschen. Die wäßrigen Phasen werden einmal mit 100 ml Ethylacetat nachextrahiert. Die vereinigten organischen Phasen werden im Vakuum zur Trockene eingedampft ; der Rückstand wird an Kieselgel chromatographiert (Laufmittel : Dichlormethan/Aceton 6:4) . Man erhält 17.14 g ( 83 % d. Th. ) Produkt als festen, farblosen Schaum.

| Elementaranalyse : | | | | |
|---|---|---|---|---|
| Ber.: | C 70.85 | H 6.77 | N 2.85 | S 6.52 |
| Gef.: | C 70.68 | H 6.91 | N 2.74 | S 6:42 |

### Beispiel 5

### a) N-Methyl-S-(4,4'-dimethoxy-trityl)-L-homocystein

Man legt 7.5 g (0.046 mol) N-Methyl-L-methionin (Titelverbindung aus Beispiel 1 b) vor und kondensiert unter MeOH/Trockeneis-Kühlung 200 ml Ammoniak ein. Bei -35°C werden portionsweise über eine Stunde 5.2 g (0.23 mol) Natrium zugegeben und zwei Stunden nachgerührt. Dann werden portionsweise 9.7 g (0.18 mol) Ammoniumchlorid zugegeben, die Kühlung entfernt und über Nacht mit Stickstoff der Ammoniak ausgetrieben. Man gibt 17.3 g (0.054 mol) Bis(4-Methoxyphenyl)-phenyl-methanol (DMT) zu. Unter Eiskühlung werden dann 50 ml Dichlormethan und 90 ml Trifluoressigsäure zugegeben. Man rührt eine Stunde bei RT und engt zur Trockne ein. Der Rückstand wird in 200 ml Wasser suspendiert und mit Natronlauge auf pH 13 gebracht. Nach einer Stunde Nachrühren saugt man ab und suspendiert den Feststoff in 500 ml Wasser. Durch Zugabe von Citronensäure wird auf pH 4 eingestellt. Es werden 600 ml MTBE zugegeben und 30 Minuten gerührt. Man saugt ab und wäscht zweimal mit je 100 ml MTBE. Der Rückstand wird mit 100 ml Dichlormethan versetzt, 20 ml Ethanol zugegeben und anschließend 500 ml MTBE zugetopft. Eine Stunde nachgerührt, abgesaugt, mit MTBE gewaschen. Nach Trocknen erhält man 13.92 g ( 67 % d. Th. ) eines farblosen Feststoffs.

| Elementaranalyse : | | | | |
|---|---|---|---|---|
| Ber.: | C 69.15 | H 6.47 | N 3.10 | S 7.10 |
| Gef.: | C 68.98 | H 6.63 | N 3.01 | S 7.02 |

### b) N-(9H-Fluoren-9-ylmethyloxycarbonyl)-N-methyl-S-(4,4'-dimethoxytrityl)-L-homocystein

18.97 g (0.042 mol) N-Methyl-S-4,4'dimethoxytrityl-L-homocystein (Titelverbindung aus Beispiel 5a ) werden in 90 ml Wasser und 100 ml THF suspendiert und 12.58 g (0.12 mol) Natriumcarbonat zugegeben. Bei 10 °C werden 14.88 g (0.044 mol) Fluorenylmethylsuccinimidylcarbonat, gelöst in 50 ml THF zugetropft und drei Stunden bei 10°C nachgerührt. Dann wird mit 80 ml Wasser und 130 ml Ethylacetat versetzt, zehn Minuten bei Raumtemperatur gerührt und mit Citronensäure auf pH 4 gebracht. Die organische Phase wird zweimal mit je 100 ml Wasser und einmal mit 100 ml Kochsalzlösung gewaschen. Die wäßrigen Phasen werden einmal mit 100 ml Ethylacetat nachextrahiert. Die vereinigten organischen Phasen werden im Vakuum zur Trockene eingedampft; der Rückstand wird an Kieselgel chromatographiert (Laufmittel : Dichlormethan/Aceton 6:4) . Man erhält 22.92 g ( 81 % d. Th. ) Produkt als festen, farblosen Schaum.

| Elementaranalyse : | | | | |
|---|---|---|---|---|
| Ber.: | C 73.08 | H 5.83 | N 2.08 | S 4.76 |
| Gef.: | C 72.95 | H 5.94 | N 2.01 | S 4.68 |

### Beispiel 6

### Fmoc-(N-CH₃)Hcy(Trt)-Tyr(tBu)-D-Trp(Boc)-Lys(Boc)-Thr(tBu)-CITrt-Festphase

Das wie in WO 01/44177 (example 1, steps 1-3) beschrieben hergestellte Festphasengeträgerte Peptid Fmoc-Tyr(tBu)-D-Trp(Boc)-Lys(Boc)-Thr(tBu)-CITrt-Festphase wurde behandelt mit 5% Piperidin in 1:1 NMP/DCM (75 mL) für 10 min gefolgt von einer Umsetzung mit 20% Piperidin in NMP (75 mL) für 15 min. Die Festphase wurde nacheinander mit NMP (3 x 75 mL x 1 min) und DCM (3 x 75 mL x 1 min) gewaschen. Eine an einer kleinen Probe der Festphase durchgeführe Ninhydrin-Analyse zeigte das Ende der Reaktion, und die Festphase wurde mit NMP (75 mL) gewaschen. Ein kleiner Teil des Trägerharzes wurde mit HFIPA behandelt und per HPLC analysiert (s. HPLC Method 1 bei Diatide). Der Peak für Fmoc-Tyr(tBu)-D-Trp(Boc)-Lys(Boc)-Thr(tBu)-OH bei 21.7 min wurde nicht detektiert, aber ein Peak bei 12.7 min für H-Tyr(tBu)-D-Trp(Boc)-Lys(Boc)-Thr(tBu)-OH. In einem separaten Behältnis wurden N-α-Fmoc-N-α-methyl-S-trityl-homocysteine (9.20 g, 15 mmol), HATU-Reagenz (5.70 g, 15 mmol), ind HOAt (2.04 g, 15 mmol) in 50 mL NMP gelöst. DIEA (6.96 mL, 40 mmol) wurde zu der Lösung des geschützten N-Methylhomocysteins gegeben. Es wurde für 1 min gerührt und die Mischung zu dem Festphasen-Ansatz gegeben. Die Reaktion wurde für 4 h unter einem leichten Argonstrom geschüttelt. Die Lösung wurde abgetrennt und die Festphase nacheinander mit NMP (3 x 75 mL x 1 min) und DCM (3 x 75 mL x 1 min) gewaschen. Eine an einer kleinen Probe der Festphase durchgeführe Ninhydrin-Analyse zeigte das Ende der Reaktion. Ein kleiner Teil des Trägerharzes wurde mit HFIPA behandelt und per HPLC analysiert (s. HPLC Method 1 bei Diatide). Der Peak für H-Tyr(tBu)-D-Trp(Boc)-Lys(Boc)-Thr(tBu)-OH bei 12.7 min wurde nicht detektiert, aber ein Peak bei 26.7 minutes für Fmoc-(N-CH₃)Hcy(Trt)-Tyr(tBu)-D-Trp(Boc)-Lys(Boc)-Thr(tBu)-OH.

## Patentansprüche

1. Verbindungen der allgemeinen Formel I 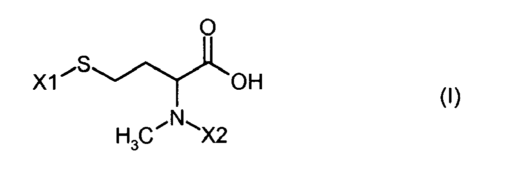 worin
X1 für eine Schwefel-Schutzgruppe und
X2 für eine Stickstoff-Schutzgruppe steht.

2. Verbindungen nach Anspruch 1, **dadurch gekennzeichnet, dass**
X1 für Benzyl (Bn), 4-Methoxybenzyl (Mob), Diphenylmethyl, Bis(4-methoxyphenyl)methyl, 4,4'-Dimethoxytriphenylmethyl (DMT), Triphenylmethyl (Trityl), Methoxymethyl (MOM), 9H-Fluoren-9-ylmethyl oder tert-Butylsulfid steht.

3. Verbindungen nach Anspruch 1, **dadurch gekennzeichnet, dass**
X2 für 9H-Fluoren-9-ylmethyloxycarbonyl (Fmoc), 2,7-Dibrom-9H-fluoren-9-ylmethyloxy-carbonyl, tert-Butyloxycarbonyl (Boc), Benzyloxycarbonyl (Cbz o. Z), Trifluoracetyl, 2,2,2-Trichloroethyloxycarbonyl (Troc), 2-Trimethylsilylethyloxycarbonyl (Teoc) oder 2-Trimethylsilylethylsulfonyl steht.

4. Verfahren zur Herstellung von Verbindungen der allgemeinen Formel I 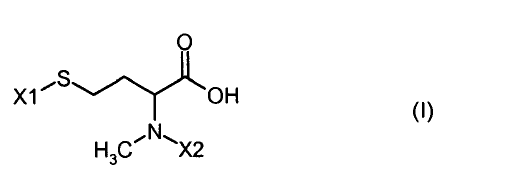 worin
X1 für eine Schwefel-Schutzgruppe und
X2 für ein Wasserstoffatom oder für eine Stickstoff-Schutzgruppe steht,
**dadurch gekennzeichnet, dass** das Oxazolidinon der Formel II 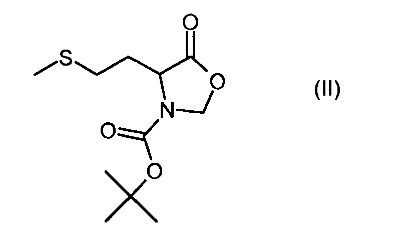 mit einem Reduktionsmittel in Gegenwart von Säure zum N-Methylmethionin der Formel III 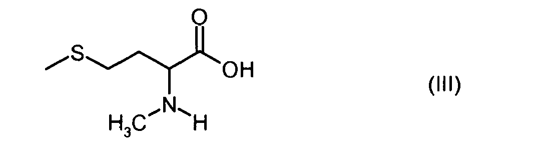 geöffnet wird und anschließend in an sich bekannter Weise eine Schwefel-Schutzgruppe sowie gegebenenfalls eine Stickstoff-Schutzgruppe eingeführt werden.

5. Verfahren nach Anspruch 4, **dadurch gekennzeichnet, dass** als Reduktionsmittel Alkylsilane verwendet werden.

6. Verfahren nach Anspruch 4, **dadurch gekennzeichnet, dass** als Säuren Trifluoressigsäure, Pentafluorpropionsäure, Trifluormethylsulfonsäure oder Methansulfonsäure verwendet wird.

7. Verwendung von Verbindungen der allgemeinen Formel I 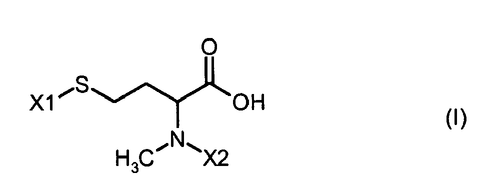 worin
X1 für eine Schwefel-Schutzgruppe und
X2 für ein Wasserstoffatom oder für eine Stickstoff-Schutzgruppe steht,
zum Aufbau von N-Methyl-Homocystein enthaltenden Peptiden und Peptidzwischenstufen.

8. Verwendung von Verbindungen der allgemeinen Formel I 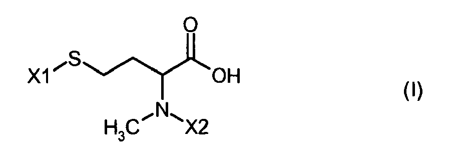 worin
X1 für eine Schwefel-Schutzgruppe und
X2 für ein Wasserstoffatom oder für eine Stickstoff-Schutzgruppe steht,
zur Herstellung von Fmoc-(N-CH₃)Hcy(Trt)-Tyr(tBu)-D-Trp(Boc)-Lys(Boc)-Thr(tBu)-OH.

9. Verwendung von Verbindungen der allgemeinen Formel I 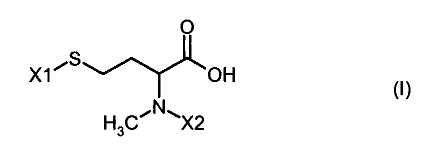 worin
X1 für eine Schwefel-Schutzgruppe und
X2 für ein Wasserstoffatom oder für eine Stickstoff-Schutzgruppe steht,
zur Herstellung von cyclo-Tyr-D-Trp-Lys-Thr-Phe-(N-CH₃)Hcy.

10. Verwendung von Verbindungen der allgemeinen Formel I 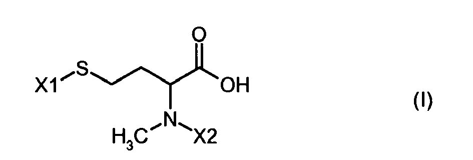 worin
X1 für eine Schwefel-Schutzgruppe und
X2 für ein Wasserstoffatom oder für eine Stickstoff-Schutzgruppe steht,
zur Herstellung von cyclo-Tyr-D-Trp-Lys-Thr-Phe-(N-CH₃)Hcy(CH₂CO-β-Dap-Phe(4-NH₂)-Cys-Thr-Ser).

## Claims

1. Compounds of the general formula I 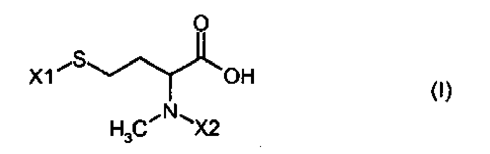 wherein
X1 represents a sulphur protecting group, and
X2 represents a nitrogen protecting group.

2. The compounds according to claim 1, **characterized in that**
X1 represents benzyl (Bn), 4-methoxybenzyl (Mob), diphenylmethyl, bis-(4-methoxyphenyl)methyl, 4,4'-dimethoxytriphenylmethyl (DMT), triphenylmethyl (trityl), methoxymethyl (MOM), 9H-fluoren-9-ylmethyl or tert-butylsulphide.

3. The compounds according to claim 1, **characterized in that**
X2 represents 9H-fluoren-9-ylmethyloxycarbonyl (Fmoc), 2,7-dibromo-9H-fluoren-9-ylmethyloxycarbonyl, tert-butyloxycarbonyl (Boc), benzyloxycarbonyl (Cbz or Z), trifluoroacetyl, 2,2,2-trichloroethyloxycarbonyl (Troc), 2-trimethylsilylethyloxycarbonyl (Teoc) or 2-trimethylsilylethylsulphonyl.

4. A method for the preparation of compounds of the general formula I 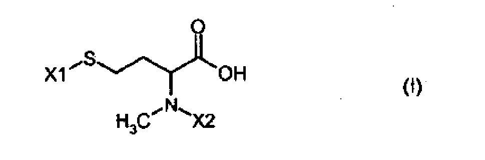 wherein
X1 represents a sulphur protecting group, and
X2 represents a hydrogen atom or a nitrogen protecting group,
**characterized in that** the oxazolidinone of the formula II 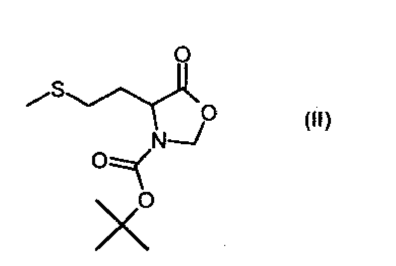 is opened with a reducing agent in the presence of acid to give N-methylmethionine, 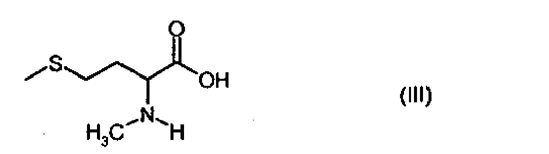 and a sulphur protecting group and optionally a nitrogen protecting group are subsequently introduced in a manner known per se.

5. The method according to claim 4, **characterized in that** alkylsilanes are used as the reducing agent.

6. The method according to claim 4, **characterized in that** trifluoroacetic acid, pentafluoropropionic acid, trifluoromethylsulphonic acid or methane sulphonic acid are used as the acids.

7. The use of compounds of the general formula I 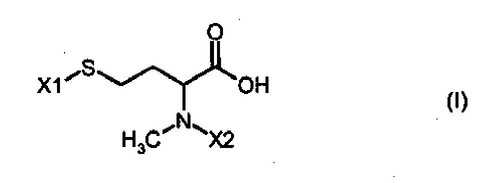 wherein
X1 represents a sulphur protecting group, and
X2 represents a hydrogen atom or a nitrogen protecting group,
for the construction of N-methylhomocysteine-containing peptides and peptide intermediates.

8. The use of compounds of the general formula I 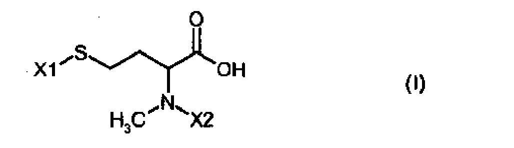 wherein
X1 represents a sulphur protecting group, and
X2 represents a hydrogen atom or a nitrogen protecting group,
for the preparation of Fmoc-(N-CH₃)Hcy(Trt)-Tyr(tBu)-D-Trp(Boc)-Lys(Boc)-Thr(tBu)-OH.

9. The use of compounds of the general formula I 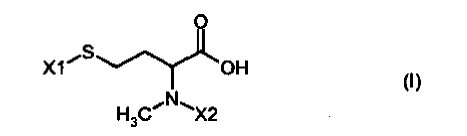 wherein
X1 represents a sulphur protecting group, and
X2 represents a hydrogen atom or a nitrogen protecting group,
for the preparation of cyclo-Tyr-D-Trp-Lys-Thr-Phe-(N-CH₃)Hcy.

10. The use of compounds of the general formula I 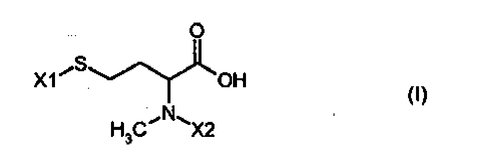 wherein
X1 represents a sulphur protecting group, and
X2 represents a hydrogen atom or a nitrogen protecting group,
for the preparation of cyclo-Tyr-D-Trp-Lys-Thr-Phe-(N-CH₃)Hcy(CH₂CO-β-Dap-Phe(4-NH₂)-Cys-Thr-Ser).

## Revendications

1. Composés de la formule générale I 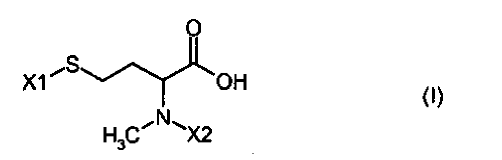 dans laquelle
X1 représente un groupe de protection du soufre et
X2 représente un groupe de protection de l'azote.

2. Composés selon la revendication 1, **caractérisés en ce que**
X1 représente le benzyle (Bn), 4-méthoxybenzyle (Mob), diphénylméthyle, bis(4-méthoxyphényl)méthyle, 4,4'-diméthoxytriphénylméthyle (DMT), triphénylméthyle (trityle), méthoxyméthyle (MOM), 9H-fluorène-9-ylméthyle ou tert-butylsulfide.

3. Composés selon la revendication 1, **caractérisés en ce que**
X2 représente le 9H-fluorène-9-ylméthyloxycarbonyle (Fmoc), 2,7-dibromo-9H-fluorène-9-ylméthyloxycarbonyle, tert-butyloxycarbonyle (Boc), benzyloxycarbonyle (Cbz ou Z), trifluoracétyle, 2,2,2-trichloroéthyloxycarbonyle (Troc), 2-triméthylsilyléthyloxycarbonyle (Teoc) ou le 2-triméthylsilyléthylsulfonyle.

4. Procédé pour la fabrication de composés de la formule générale I 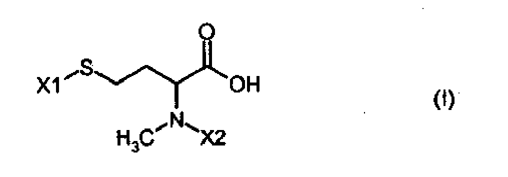 dans laquelle
X1 représente un groupe de protection du soufre et
X2 représente un atome d'hydrogène ou un groupe de protection de l'azote, **caractérisé en ce que** l'oxazolidinone de la Formule II 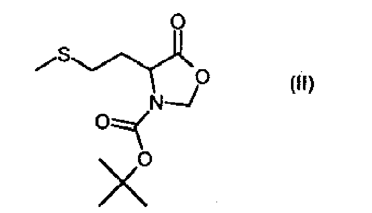 est ouvert avec un agent réducteur en présence d'acide vers le N-méthylméthionine de la Formule III, 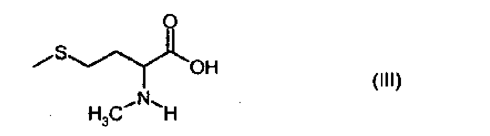 puis sont introduits d'une manière en fait connue un groupe de protection du souffre ainsi que, le cas échéant, un groupe de protection de l'azote.

5. Procédé selon la revendication 4, **caractérisé en ce que** l'on utilise comme agent réducteur de l'alkylsilane.

6. Procédé selon la revendication 4, **caractérisé en ce que** l'on utilise comme acides de l'acide trifluoro-acétique, de l'acide pentafluoro-propionique, de l'acide trifluorométhylsulfonique ou de l'acide méthanosulfonique.

7. Utilisation de composés de la formule générale I 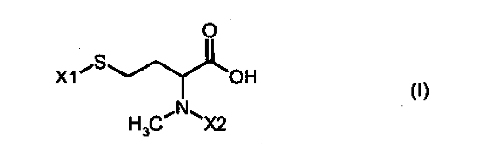 dans laquelle
X1 représente un groupe de protection du soufre et
X2 représente un atome d'hydrogène ou un groupe de protection de l'azote, pour l'établissement de peptides et étages intermédiaires de peptides contenant de la n-méthyl-homocystéine.

8. Utilisation de composés de la formule générale I 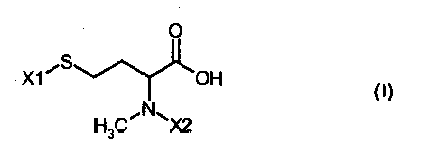 dans laquelle
X1 représente un groupe de protection du soufre et
X2 représente un atome d'hydrogène ou un groupe de protection de l'azote, pour la fabrication de Fmoc-(N-CH₃)Hcy(Trt)-Tyr(tBu)-D-Trp(Boc)-Lys(Boc)-Thr(tBu)-OH.

9. Utilisation de composés de la formule générale I 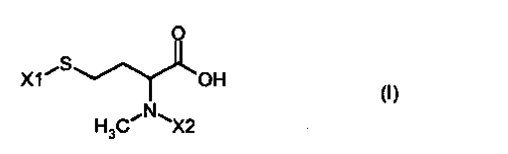 dans laquelle
X1 représente un groupe de protection du soufre et
X2 représente un atome d'hydrogène ou un groupe de protection de l'azote, pour la fabrication de cyclo-Tyr-D-Trp-Lys-Thr-Phe-(N-CH₃)Hcy.

10. Utilisation de composés de la formule générale I 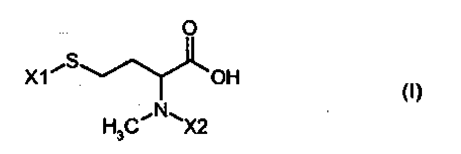 dans laquelle
X1 représente un groupe de protection du soufre et
X2 représente un atome d'hydrogène ou un groupe de protection de l'azote,
pour la fabrication de cyclo-Tyr-D-Trp-Lys-Thr-Phe-(N-CH₃)Hcy(CH₂CO-β-Dap-Phe(4-NH₂)-Cys-Thr-Ser).
